(19) Europäisches Patentamt
European Patent Office
Office européen des brevets

(11) Publication number: **0 188 781**
**B1**

# (12) EUROPEAN PATENT SPECIFICATION

(45) Date of publication of patent specification: **24.10.90**

(21) Application number: **85116269.3**

(22) Date of filing: **19.12.85**

(51) Int. Cl.$^5$: **C 07 D 498/04,** A 61 K 31/535 // (C07D498/04, 265:00, 205:00)

## (54) 1-Oxa-1-dethia-cephalosporin compounds and antibacterial agent comprising the same.

(30) Priority: **28.12.84 US 687096**

(43) Date of publication of application:
**30.07.86 Bulletin 86/31**

(45) Publication of the grant of the patent:
**24.10.90 Bulletin 90/43**

(84) Designated Contracting States:
**CH DE FR GB IT LI NL**

(56) References cited:
**EP-A-0 099 580**
**GB-A-2 001 071**

(73) Proprietor: **MERCK & CO. INC.**
**126, East Lincoln Avenue P.O. Box 2000**
**Rahway New Jersey 07065-0900 (US)**

(73) Proprietor: **MEIJI SEIKA KAISHA LTD.**
**4-16 Kyobashi 2-chome**
**Chuo-ku Tokyo 104 (JP)**

(72) Inventor: **Shibahara, Seiji**
**3-18-30, Tsukushino**
**Machida-shi Tokyo (JP)**
Inventor: **Okonogi, Tsuneo**
**2-5-11-402, Susukino Midori-ku**
**Yokohama-shi Kanagawa-ken (JP)**
Inventor: **Murai, Yasushi**
**2-37-19, Futaba**
**Yokosuka-shi Kanagawa-ken (JP)**
Inventor: **Fukatsu, Shunzo**
**1-13, Ichigaya-Tamachi**
**Shinjuku-ku Tokyo (JP)**
Inventor: **Niida, Taro**
**127, Naka-Kibohgaoka Asahi-ku**
**Yokohama-shi Kanagawa-ken (JP)**
Inventor: **Wakazawa, Tadashi**
**2602-53, Kamariya-cho Kanazawa-ku**
**Yokohama-shi Kanagawa-ken (JP)**
Inventor: **Christensen, Burton G.**
**770 Anderson Avenue Apt. 19H**
**Cliffside New Jersey 07010 (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(74) Representative: Abitz, Walter, Dr.-Ing. et al
Abitz, Morf, Gritschneder, Freiherr von
Wittgenstein Postfach 86 01 09
D-8000 München 86 (DE)

**Description**

This invention relates to a novel antibacterial 1-oxa-1-dethiacephalosporin compound.

We, the present inventors, have made extensive researches in an attempt to seek for and to synthesize such a new cephalosporin compound which exhibits a wide range of antibacterial spectrum and which is active against a variety of resistant bacteria. As a result, the present inventors have now succeeded in preparing 1-oxa-1-dethiacephalosporin compounds which are effective as an antibacterial agent in therapeutic treatment of animals including man.

Thus, the present inventors have succeeded in the synthesis of a 1-oxa-1-dethiacephalosporin compound of the general formula (I)

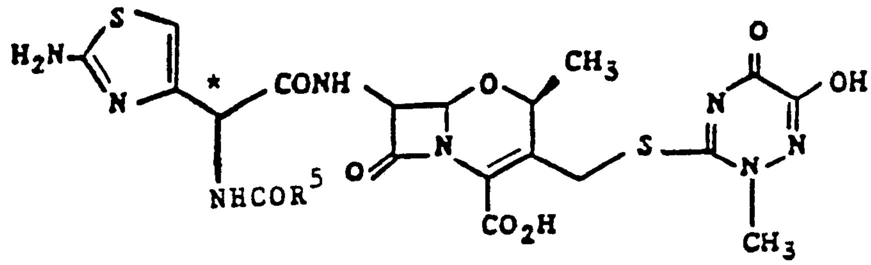

where $R^5$ is a hydrogen atom, a 4-ethyl-2,3-dioxo-piperazin-1-yl group or is selected from the groups consisting of:

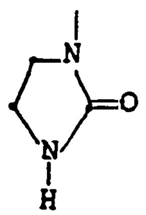 , 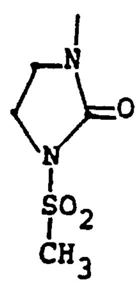 , 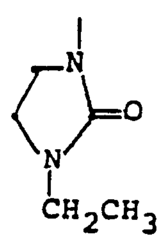 , 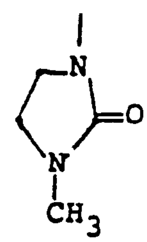 , 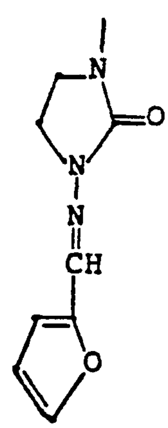

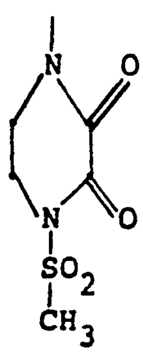 , 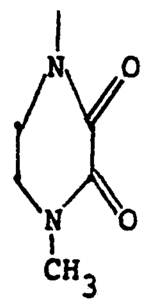 or 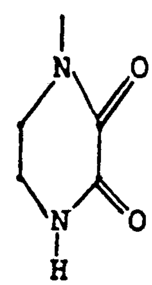

and the carbon atom to which the asterisk is attached shows (R)-configuration or (S)-configuration or a mixture of these configurations; and a pharmaceutically acceptable hydrate, salt or ester thereof. And, we have found that the 1-oxo-1-dethia-cephalosporin compounds bearing a (2,5-dihydro-6-hydroxy-2-methyl-5-oxo-1,2,4-triazin-3-yl) thiomethyl group at the 3-position thereof show a wide range of antibacterial activity against gram-positive and gram-negative bacteria, including a variety of resistant bacteria, and that these compounds exhibit an excellent activity as an antibacterial agent when administered intramuscularly or intravenously to the animals.

Japanese patent application first publication "KOKAI" No. 154980/80 discloses such cephalosporin compounds which have at the 3-position thereof a (2,5-dihydro-6-hydroxy-2-methyl-5-oxo-1,2,4-triazin-3-yl) thiomethyl group, but this first publication has no description in respect of the 1-oxa-1-dethia-cephalosporin compounds. The compounds of the formula (I) mentioned above are thus novel compounds.

EP—A—0 099 580 and GB—A—2 001 071 refer to 1-oxa-1-dethia-cephalosporin derivatives which differ from the compounds of the present invention in having other substituents in the 7-position.

It is known that generally, such 1-oxa-1-dethia-cephalosporin compounds having a 2-aminothiazol-4-acetic acid derivative as a substituent on the 7-amino group thereof are inferior in the antibacterial activity to the corresponding cephalosporins (R. B. Morin, M. Gorman "Chemistry and Biology of B-lactam Antibiotics" *2,* pages 1—98 (1982), Academic Press, New York). While, the present inventors have found that though the compound of the general formula (I) according to this invention has a 2-aminothiazol-4-acetic acid derivative as a substituent on the 7-amino group, the new compound of this invention exhibits an excellent inhibitory activity to *Staphylococcus faecalis* against which the corresponding cephalosporins (as referred to in said Japanese patent application first publication "KOKAI" No. 154980/80) are ineffective. In this way, the invention have been accomplished.

According to a first aspect of this invention, therefore, there is provided a 1-oxa-1-dethiacephalosporin compound of the general formula (I) mentioned above, and a pharmaceutically acceptable hydrate, salt or ester thereof.

Typical examples of the compounds of the general formula (I) include the following:

No. 1 (6R,7R)-7-[2-(2-aminothiazol-4-yl)-2-(4-ethyl-2,3-dioxo-1-piperazinecarboxamido) acetamidol]-(2S)-2-methyl-3-(2,5-dihydro-6 hydroxy-2-methyl-5-oxo-1,2,4-triazin-3-yl) thio methyl-1-oxa-1-dethia-ceph-3-em-4-carboxylic acid of the formula

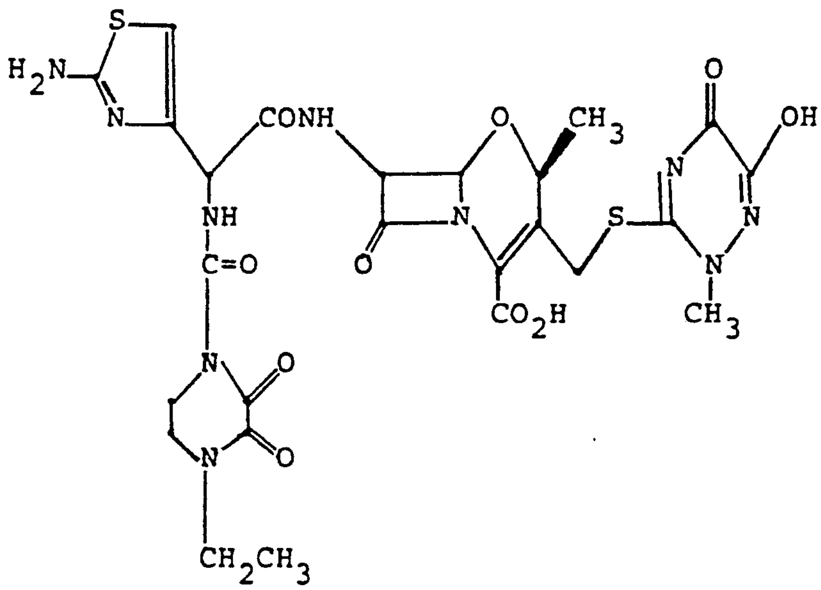

No. 2 (6R,7R)-7-[2-(2-aminothiazol-4-yl)-2-(formamido)acetamido]-(2S)-2-methyl-3-(2,5-dihydro-6-hydroxy-2-methyl-5-oxo-1,2,4-triazin-3-yl) thiomethyl-1-oxa-1-dethia-ceph-3-em-4-carboxylic acid of the formula

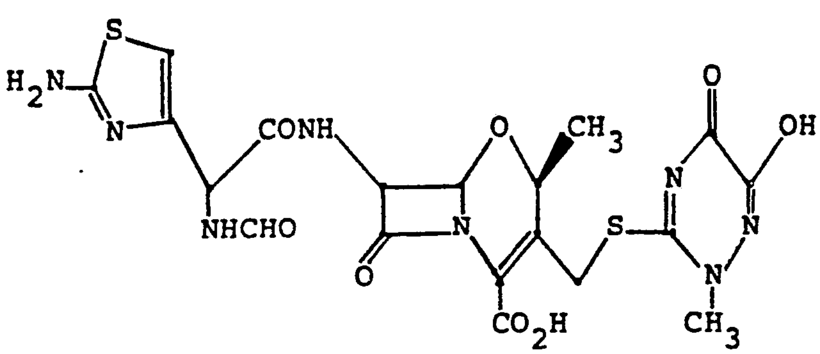

The above-mentioned compound numbers are referred to in Tables and Examples given hereinafter.

The compound of the general formula (I) according to this invention includes a solvate, particularly hydrate of said compound (I). Moreover, this invention also covers a non-toxic salt and a non-toxic ester of the compound of the formula (I), and in particular, a metabolically unstable ester thereof, as will be described later. By the term "non-toxic" is meant "pharmaceutically acceptable."

The above-exemplified representatives of the novel compounds of the formula (I) according to this invention have the following advantages;

(a) As will be clear from Table I given below which shows antibacterial spectra of the test compounds No. 1 and No. 2 against a variety of gram-positive and gram-negative bacteria as determined in vitro in term of the minimum inhibitory concentration (MIC) of said test compounds against said bacteria, the compounds of this invention exhibit a high antibacterial activity against a wider range of bacteria. For comparison, MIC of Latamoxef and Ceftriaxone are also indicated in the Table 1.

As be apparent from the Table I, not only the compounds of this invention exhibit a practically effective, high antibacterial activity against a variety of bacteria, including the resistant strains thereof, but also those compounds as indicated in Examples 1 and 2 in particular, show such a remarked advantage that they are effective against *Streptococcus faecalis.* Besides, the compound of Example 1 advantageously is also effective against *Pseudomonas aeruginosa.*

Furthermore, the compound of the formula (I) according to this invention has an advantage that it provides a higher level of the antibacterial activity and a higher concentration of the compound in blood serum as well as a longer durability of the blood serum concentrate, as compared to such 1-oxa-1-dethia-cephalosporin derivatives which are disclosed in the specification of Japanese patent application first publication "KOKAI" No. 46287/84 invented by the same inventors as of the present invention.

TABLE 1
Minimum inhibitory concentration (MIC)

| Test Organism | Compound No. 1 | Compound No. 2 | Latamoxef (comparative) | Ceftriaxone (comparative) |
|---|---|---|---|---|
| Staphylococcus aureus Smith | 3.13 | 1.56 | 3.13 | 3.13 |
| Staphylococcus aureus 209 P JC—1 | 1.56 | 0.78 | 3.13 | 1.56 |
| Escherichia coli NIHJ JC-2 | 0.10 | 0.10 | 0.10 | 0.10 |
| Escherichic coli GN206 | 1.56 | 1.56 | 0.39 | 0.78 |
| Salmonella typhimurium LT-2 | 0.10 | 0.025 | 0.05 | 0.05 |
| Proteus mirabilis GN79 | 0.78 | 0.10 | 0.10 | 0.025 |
| Proteus vulgaris GN76 | 0.025 | 50 | 0.20 | 0.025 |
| Proteus rettgeri GN624 | 0.78 | 0.10 | 0.05 | 0.39 |
| Serratia marcescens No. 2 | 0.39 | 0.20 | 0.10 | 0.10 |
| Pseudomonas | | | | |
| aeruginosa M83833 | 3.13 | 100 | 6.25 | 50 |
| „ M83829 | 1.56 | 50 | 12.5 | 12.5 |
| „ E-2 | 3.13 | 100 | 6.25 | 100 |
| Streptococcus faecalis ATCC8043 | 50 | 12.5 | 25 | 3.13 |
| „ W-73 | 25 | 100 | 100 | 100 |
| „ W-74 | 6.25 | 50 | 100 | 100 |
| „ W-75 | 3.13 | 6.24 | 100 | 1.56 |

(b) The novel compounds of this invention have such a pharmacological merit that they show an enhanced maximum of the compound concentration in serum and a longer durability of the compound concentration in serum after the administration.

In order to demonstrate experimentally these merits the novel compound was given at a dosage of 25 mg/kg subcutaneously to mice (three mice in each group, average body weight 20 g), and blood was sampled regularly from the animals.

For the serum which was separated from the blood samples, the concentration in the serum of the compound administered was bioassayed using *Escherichia coli* as assay microorganism.

The test results obtained are shown in Table 2 below:

TABLE 2

| Compound Tested | Concentration in serum (mcg/ml) 1/12 | 1/4 | 1/2 | 1 hour |
|---|---|---|---|---|
| Compound No. 1 | 24 | 44 | 25 | 10 |

As demonstrated by the foregoing tests (a) and (b), the novel compound of the formula (I) according to this invention has remarkably excellent properties as the antibacterial agent, so that it is a useful antibiotic which can be administered orally or parenterally for curative or preventative treatment of bacterial infections in mammalian animals, including man. Improved duration of the compound concentration in blood as described above is an important factor by which the antibacterial activity in vitro can be developed in vivo.

According to a second aspect of this invention, therefore, there is provided an antibacterial agent which comprises at least one of a 1-oxa-1-dethia-cephalosporin compound represented by the aforesaid general formula (I), and a non-toxic hydrate, a non-toxic salt or a non-toxic ester of said compound as the active ingredient.

Non-toxic salt, that is, the pharmaceutically acceptable salt of the compound of general formula (I) includes conventional non-toxic salts (carboxylates) which may be formed by reaction of the carboxyl group present in said compound with a base, especially such salts with an inorganic base, for example, alkali metal salts such as sodium or potassium salt and alkaline earth metal salts such as calcium, magnesium or zinc salt; such addition salts with a basic amino acid, for example, lysine, arginine, ornithine or histidine; and such addition salts with an organic amine salt or other basic salts which will normally form a salt with cephalosporin.

Other non-toxic salts of the compound (I) according to this invention include those which may be formed by addition to the amino group or other basic group of an inorganic acid such as hydrochloric, hydrobromic, sulfuric, nitric or phosphoric acid, of an organic carboxylic acid or organic sulfonic acid such as trifluoroacetic, benzenesulfonic, methanesulfonic, maleic, tartaric or p-toluenesulfonic acid, and of an acidic amino acid such as aspartic or glutamic acid, and further they may include intermolecular or intramolecular salts.

The non-toxic esters of the compound (I) according to this invention include those of the carboxyl group present in said compound with a pharmaceutically acceptable ester-forming group. Among them are preferred such metabolically unstable esters, which carry an ester-forming group cleavable upon hydrolysis in vivo. Examples of such ester-forming group include acetoxymethyl, pivaloyloxymethyl, α-ethoxycarbonyloxyethyl, phthalidyl and phenyl groups.

For use as antibacterial agent, the compound (I) of this invention may be given orally or parenterally to adults at a unit dosage of 50 to 1500 mg and preferably of 100 to 1000 mg once to five times per day when it is administered for therapeutic treatment of bacterial infections in man. The antibacterial agent according to this invention may usually be composed of the compound of this invention in association with a pharmaceutically acceptable carrier such as a solid or liquid excipient, and it may be formulated into solid preparations such as tablets, capsules, powder and pre-treated powder, or into liquid preparations such as injectable solution, intravenous drip solution, suspension and syrup. Solid or liquid excipient used for this purpose may be any one known in this field of the art. As stated above, the preparations so formed may preferably contain the compound of this invention at an amount required for the unit dosage of the compound for adults indicated above.

The compound (I) according to this invention may be produced, for example, by reacting a 1-oxa-1-dethia-cephem compound of the general formula (IIa)

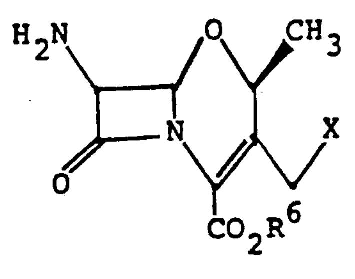

(IIa)

wherein $R^6$ is a carboxyl-protecting group, and X is a chlorine, bromine or iodine atom, with a carboxylic acid of the general formula (III)

$$R^1COOH \qquad (III)$$

wherein $R^1$ represents a residue of the general formula,

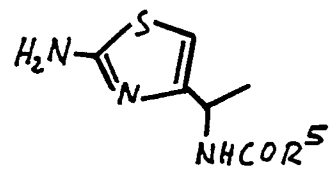

wherein the amino group in the 2-position of the thiotol ring can be protected by an amino-protecting group, or with a functional derivative thereof, to give a 7-N-acylation product of the general formula (IV)

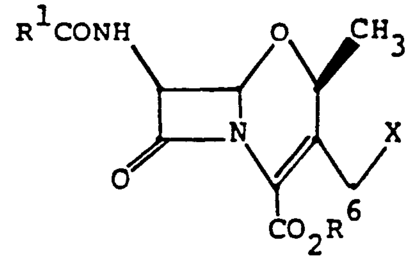

(IV)

wherein $R^1$, $R^6$ and X are as defined above, and reacting the resultant 7-N-acylated compound of the formula (IV) with 2,5-dihydro-6-hydroxy-2-methyl-5-oxo-3-mercapto-1,2,4-triazin of the formula (V)

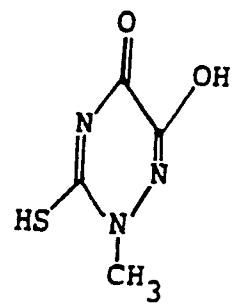

(V)

preferably in the presence of a base which acts as binding agent of hydrogen halide, for example an alkyl amine, to give a compound of the general formula (VI)

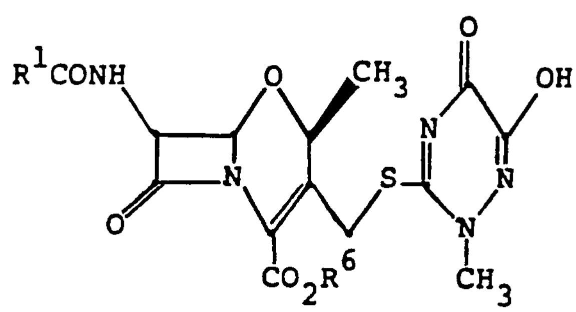

(VI)

wherein $R^1$ and $R^6$ are as defined hereinabove, followed by removal in a conventional manner of the remaining protecting groups from the resultant compound of the formula (VI) above.

The steps in the process for the synthesis of the compound of the general formula (I) from the compound of the general formula (II) are described below;

In order to introduce a desired acyl group to the 7-amino group of the compound (II), the compound of the formula (II) is at first reacted with the carboxylic acid of the formula (III) to effect the acylation on the 7-amino group, and thereby to obtain the 7-N-acylated product of the formula (IV). This acylation reaction may be carried out in a conventional manner known per se.

The carboxylic acid of the formula (III) may preferably be used in the form of a functional derivative of said carboxylic acid such as an acid halide, an active acid ester, an acid anhydride or a mixed acid anhydride thereof. The acylation reaction may also be performed using the carboxylic acid of the formula (III) which has not been activated as above, when the reaction may be carried out by a dehydration condensation method with carbodiamido or by a phosphorous oxychloride method. The acylation may generally be performed in an organic solvent which is inert to the reaction such as dioxane, tetrahydrofuran, dimethylformamide, methylene chloride, chloroform or ethyl acetate, at a temperature of −50°C to +50°C.

Next, the 7-N-acylated product of the formula (IV) is reacted with 2,5-dihydro-6-hydroxy-2-methyl-5-oxo-3-mercapto-1,2,4-triazin (V) as the reagent to introduce a desired substituent for the 2'-halogen atom, whereby to obtain the compound (VI). This replacement reaction at the 3'-position is itself a known reaction which is disclosed in the specification of Japanese patent application first publication "KOKAI" No. 154980/80. The compounds (IV) and (V) are mixed with each other in an inert organic solvent, preferably in dimethylformamide (DMF), dimethylacetoamide or dioxane, at 0°C to 80°C to effect the reaction. In case the compound of the formula (IV) where X is a chlorine atom, 1—2 equivalent of sodium iodide, (NaI) may preferably be added to the reaction system to ensure that the reaction proceeds in a higher yield.

Removal of the remaining protecting group from the compound (VI) gives the compound of the general formula (I) according to this invention. The protecting groups which are remaining in the compound (VI) are the carboxyl-protecting group on the 4-carboxyl group and the amino-protecting group on the 2-amino group of the 2-aminothiazol side chain at the 7-position of the 1-oxacepham ring. These protecting groups may be removed successively or simultaneously in a conventional manner. In case $R^6$ is a diphenylmethyl group or p-nitrobenzyl group and the 2-amino group of the 2-aminothiazol moiety has been protected with a trityl group, these protecting groups are removed simultaneously by such a method as catalytic reduction or hydrolysis with an acidic reagent. The deprotection reaction for this purpose is known and performed in a conventional manner using an acidic reagent, for example, a mineral acid such as hydrochloric acid, an organic acid such as trifluoroacetic acid, or a Lewis acid such as aluminum chloride.

The compound of the general formula (IIa) employed as a starting material for the process of producing the compound (I) of this invention may be prepared by the following reaction flow sheet:

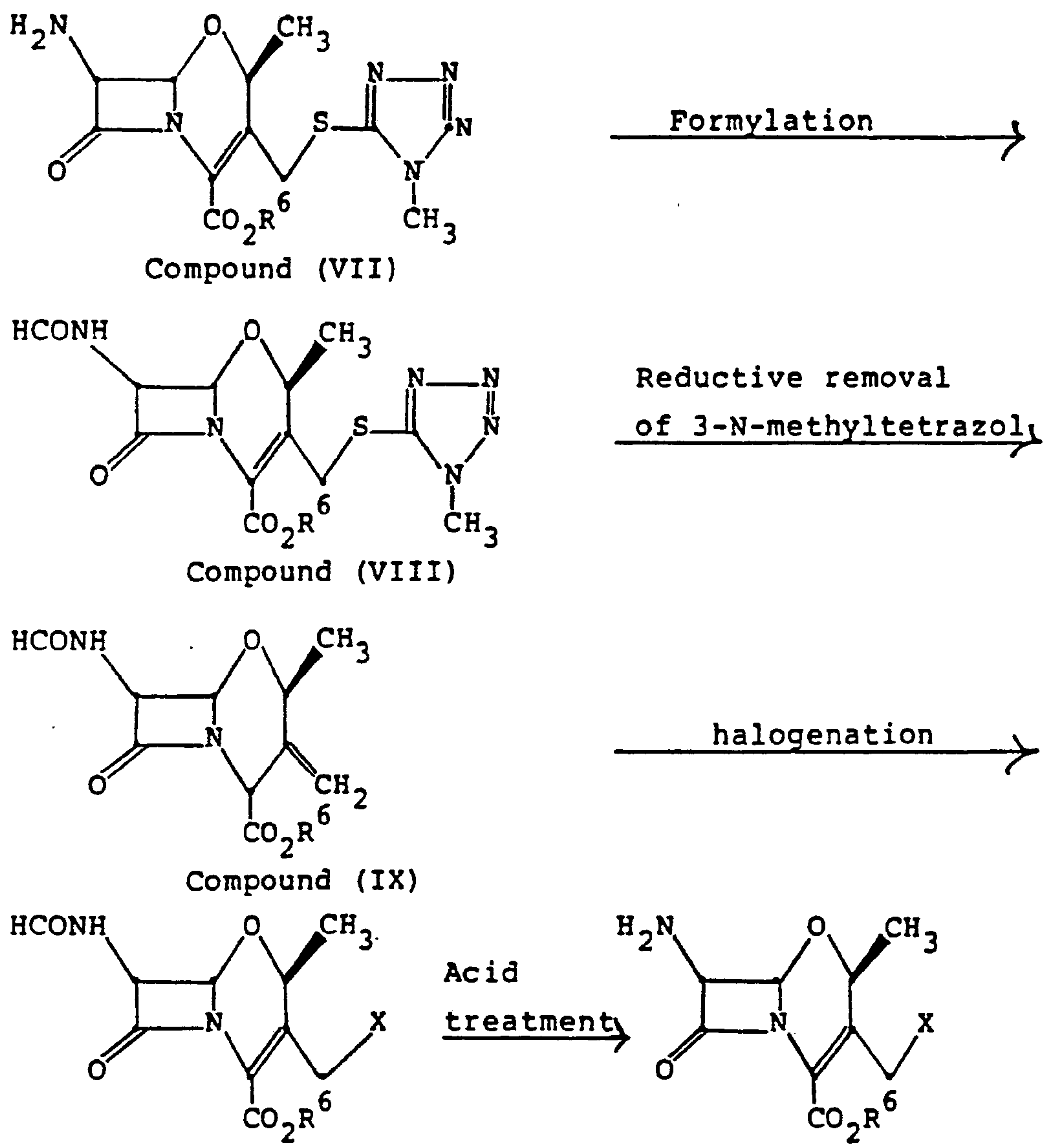


Compound (VII)

Compound (VIII)

Compound (IX)

Compound (X)

Compound (IIa)

In this reaction flow sheet, $R^6$ and x denote the same meaning as defined hereinabove.

The compound of the formula (VII) which is used as a starting material for the preparation of the compound (IIa) shown in the above reaction flow sheet may be synthesized according to the method as disclosed in Japanese patent application first publications "KOKAI" No. 46287/84 and No. 51291/84 each invented by some of the present inventors. In the formulas in the reaction flow sheet shown above, $R^6$ is a carboxyl-protecting group which may generally be used for penicillins, cephalosporins and 1-oxa-dethia-cephalosporins. These carboxyl-protecting groups may preferably be an alkyl group such as 1-butyl group, dichloroethyl group and trichloroethyl group; and an aralkyl group such as benzyl group, p-nitriobenzyl group, p-methoxybenzyl group, phenacylmethyl group and diphenylmethyl group. Among these protecting groups, preferred are ones which are easily removed under a reductive condition, for example, diphenylmethyl group and p-nitrobenzyl group.

In the first step for the preparation of the compound (IIa), the starting compound (VII) is formylated so that the 7-amino group is protected and the compound (VIII) is formed. This formylation reaction may be carried out in a conventional manner, when it is convenient to use a mixture of formic acid and acetic anhydride or an active ester of formic acid, preferably trichlorophenylester of the formula:

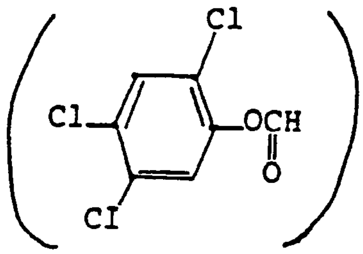

Thus, the 7-N-formylated product (VIII) is obtained. Solvents used in the formylation may be an ordinary organic solvent, particularly an inert organic solvent which may preferably be methylene chloride, chloroform, tetrahydrofuran, or dioxane.

In the next 2nd step, the 3-N-methyltetrazol group of the compound (VIII) is reductively removed to give the 3-methyleno-1-oxacepham compound of the formula (IX). The reduction may be carried out at −5°C to 50°C in an inert solvent in the presence of an organic or inorganic acid as the hydrogen source and using a metal powder such as zinc, iron, magnesium and the like. The reduction is preferably performed in methylene chloride or dioxane using zinc or magnesium in the presence of formic acid or acetic acid.

In the 3rd step, the compound (IX) is treated with a halogenating agent to give a corresponding 3-halogenomethyl compound (X) which is a novel compound. The halogenation of the compounds (IX) may be performed according to the halogenation procedure as described in "Journal of American Chemical Society", *99*, 2822 (1977), and "Tetrahedron", *39*, No. 15, 2515—2526 (1983). For the halogenation, the compound (IX) may be reacted with bromine or iodine, preferably in the presence of organic base DBU to give a 3-bromomethyl compound (X) (X=Br) and a 3-iodomethyl compound (X) (X=I), respectively. A 3-chloromethyl compound (X) (X=Cl) may be obtained by reacting the compound (IX) with phenylselenyl chloride, followed by oxidative deselenylation of the resultant 3-phenylseleno-1-oxacepham compound.

In the fourth step, the formyl group for the 7-amino-protecting group of the compound (X) is removed to give the compound of the formula (IIa). Deformylation by acid treatment is a well-known reaction, and to this end anhydrous hydrochloric acid is preferred to ensure that the deformylation can be performed selectively without affecting the other protecting group which is present in the molecule and unstable to the acid, that is, the protecting group for the 4-carboxylic group of said compound (X). The deformylation may be carried out with 2—8 equivalents of anhydrous hydrochloric acid in an inert solvent at a temperature of −5°C to +20°C. Depending on the procedure employed after the deformylation reaction, the compound of the general formula (IIa) is isolated either in the form of its free base or in the form of its hydrochloride.

Thus, according to a third aspect of this invention, there is provided, as an important intermediate product, a 1-oxa-1-dethiacephem compound of the general formula (IIb)

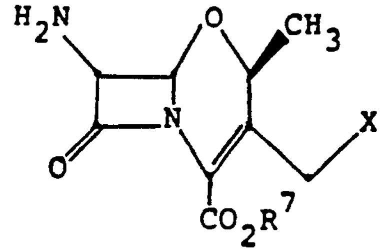

(IIb)

wherein X is a chlorine atom, a bromine atom or an iodine atom, and $R^7$ is a hydrogen atom or a carboxyl-protecting group.

This invention is now illustrated with reference to the following Examples.

Example 1

(1) Production of diphenylmethyl 7β-formamido-2β-methyl-3-(1-methyl-1H-tetrazol-5-yl)thiomethyl-1-oxa-1-dethia-3-cephem-4-carboxylate of the formula:

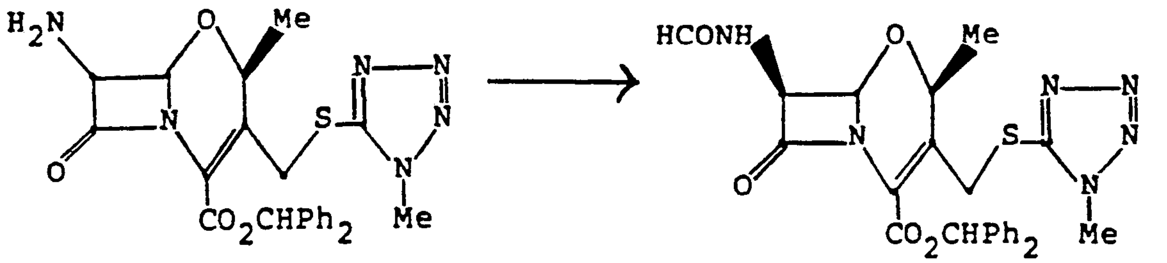

wherein Me is a methyl and Ph is a phenyl group, and also hereinafter as otherwise stated.

Diphenylmethyl 7β-amino-2β-methyl-3-(1-methyl-1H-tetrazol-5-yl)thiomethyl-1-oxa-1-dethia-3-cephem-4-carboxylate (800 mg) and 440 mg of 2,4,5-trichlorophenyl formate were dissolved in 40 ml of methylene chloride, and the solution was allowed to stand at ambient temperature for 20 hours and then concentrated. The residue was washed with ethyl acetate and recrystallized from methylene chloride to give 664 mg (yield: 79%) of the titled compound.

IR (Nujol), $_{max}$(cm$^{-1}$): 1779, 1722, 1662

NMR ($CDCl_3$), (ppm): 1.55 (3H, d, J=6.6 Hz), 3.82 (3H, s), 4.73, 4.05 (2H, ABq, J=13.7 Hz), 4.89 (1H, q, J=6.6 Hz)), 5.11 (1H, d, J=3.9 Hz), 5.76 (1H, dd, J=9.5, 6.5 Hz), 6.89 (1H, s), 7.10—7.70 (10H, m), 8.28 (1H, s).

(2) Production of diphenylmethyl 7β-formamido-2β-methyl-1-oxa-1-dethia-3-methylenecepham-4-carboxylate of the formula:

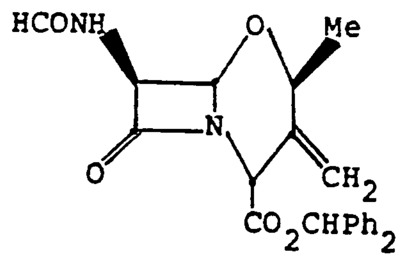

The product from the step (1) above, that is, diphenylmethyl 7β-formamido-2β-methyl-3-(1-methyl-1H-tetrazol-5-yl)thiomethyl-1-oxa-1-dethia-3-cephem-4-carboxylate (370 mg) was dissolved in 7 ml of methylene chloride, to which were added 2.0 ml of acetic acid and 170 mg of magnesium (metal). The mixture obtained was agitated at ambient temperature for 40 hours and filtered. The filtrate so obtained was concentrated.

The residue obtained was dissolved in ethyl acetate, washed with saturated aqueous sodium bicarbonate and then with water, dried over $MgSO_4$ and concentrated.

The residue was purified by column chromatography on silica gel, to give 166 mg (58%) of the titled compound.

IR ($CHCl_3$), $_{max}$(cm$^{-1}$): 1780, 1741, 1695

NMR ($CDCl_3$), (ppm): 1.37 (3H, d, J=6.3 Hz), 4.37 (1H, q, J=6.3 Hz), 5.13 (1H, s), 5.29 (1H, s), 5.36 (1H, s), 5.41 (1H, d, J=3.5 Hz), 5.61 (1H, dd, J=10.1, 3.5 Hz), 6.20 (1H, d, J=10.1 Hz), 6.85 (1H, s), 7.10—7.40 (10H, m), 8.19 (1H, s).

(3) Production of diphenylmethyl 7β-formamido-2β-methyl-3-chloromethyl-1-oxa-1-dethia-3-cephem-4-carboxylate of the formula:

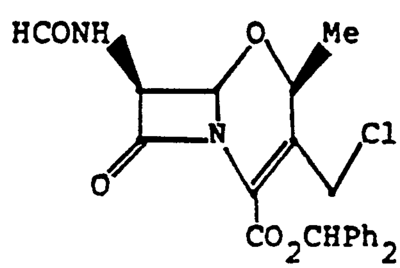

The product from the step (2) above, that is, diphenylmethyl 7β-formamido-2β-methyl-1-oxa-1-dethia-3-methylenecepham-4-carboxylate (300 mg) and 280 mg of phenylselenyl chloride were dissolved in 8 ml of methylene chloride, and the solution was agitated at ambient temperature for 5 hours. 40% Aqueous peracetic acid (300 mg) was added under ice-cooling to said solution, followed by agitation at ambient temperature for 30 minutes.

The resultant reaction solution was washed with saturated aqueous sodium bicarbonate, with aqueous sodium thiosulfate and then with water, dried over $MgSO_4$ and concentrated.

The residue was purified by column chromatography on silica gel to afford 250 mg (77%) of the titled compound.

IR ($CHCl_3$), $_{max}(cm^{-1})$: 1797, 1723, 1697

NMR ($CDCl_3$), (ppm): 1.51 (3H, d, J=6.6 Hz), 4.11, 5.16 (2H, ABq, J=12.5 Hz), 4.78 (1H, q, J=6.6 Hz), 5.12 (1H, d, J=3.7 Hz), 5.77 (1H, dd, J=9.9, 3.7 Hz), 6.28 (1H, d, J=9.9 Hz), 6.90 (1H, s), 7.10—7.60 (10H, m), 8.24 (1H, s).

(4) Production of diphenylmethyl 7β-amino-2β-methyl-3-chloromethyl-1-oxa-1-dethia-3-cephem-4-carboxylate of the formula:

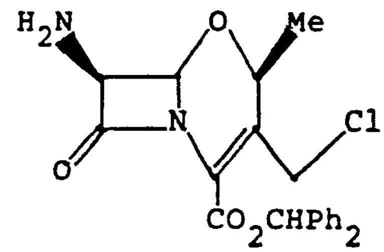

The product from the step (3) above, that is, diphenylmethyl 7β-formamido-2β-methyl-3-chloromethyl-1-oxa-1-dethia-3-cephem-4-carboxylate (200 mg) was dissolved in 2 ml of methanol. The solution was admixed with 75 µl of a solution of 6N-HCl in dioxane, agitated at ambient temperature for 30 minutes and concentrated.

The residue was washed with ether and dried to give 200 mg (a quantitative yield) hydrochloride of the titled compound.

The free base of the title compound shows the following physico-chemical properties:

IR ($CHCl_3$), $_{max}(cm^{-1})$: 1783, 1720

NMR ($CDCl_3$), (ppm): 1.52 (3H, d, J=6.7 Hz), 1.85 (2H, s), 4.11, 5.15 (2H, ABq, J=12.8 Hz), 4.49 (1H, d, J=4.0 Hz), 4.77 (1H, q, J=6.8 Hz), 5.04 (1H, d, J=4.0 Hz), 6.90 (1H, s), 7.10—7.60 (10H, m).

(5) Production of disodium (6R,7R) - 7 - [2 - (2 - aminothiazol - 4 - yl) - 2 - (4 - ethyl - 2,3 - dioxo - 1 - piperazine - carboxamido)acetamido] - (2S) - 2 - methyl - 3 - (2,5 - dihydro - 6-hydroxy - 2 - methyl - 5 - oxo - 1,2,4 - triazin - 3 - yl)thiomethyl - 1 - oxa - 1 - dethia - 3 - cephem - 4 - carboxylate (disodium salt of compound No. 1)

(1) 227 Mg of diphenylmethyl 7β-amino-2β-methyl-3-chloromethyl-1-oxadethia-3-cephem-4-carboxylate and 341 mg of potassium DL-2-(4-ethyl-2,3-dioxo-1-piperazinecarboxamido)-2-(tritylaminothiazol-4-yl) acetate were dissolved in 6 ml of methylene chloride. To the solution were added 160 µl of pyridine and 65 µl of phosphorous oxychloride at −20°C, and the mixture was agitated at the same temperature as above for 30 minutes and further under ice-cooling for 30 minutes.

The reaction solution was washed with acidified water (pH 3.0), with saturated aqueous sodium bicarbonate and then with water, dried over $MgSO_4$ and concentrated.

The diastereomers so obtained, which were composed of two isomers having Rf value 0.43 and 0.26, respectively (silica gel chromatography; developed with ethyl acetate), were separated from each other and purified by column chromatography on silica gel developed with a development solvent of toluene-ethyl acetate (1:2).

There were thus obtained 172 mg of the isomer (corresponding to Rf=0.43) and 149 mg of the isomer (corresponding to Rf 0.26) of diphenylmethyl 7β - [2 - (4 - ethyl - 2,3 - dioxo - 1 - piperazine-carboxamido) - 2 - (2 - tritylaminothiazol - 4 - yl)acetamido] - 2β - methyl - 3 - chloromethyl - 1 - oxa - 1 - dethia - 3 - cephem - 4 - carboxylate of the formula

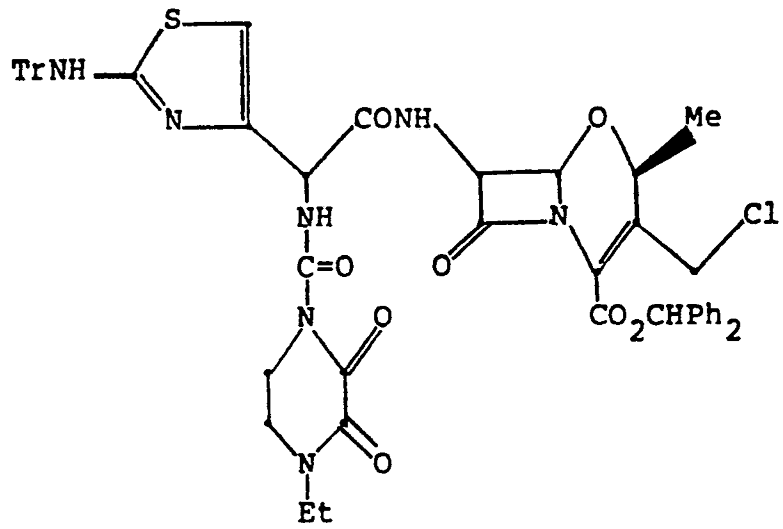

The compound of Rf 0.43 shows:

NMR ($CDCl_3$), (ppm): 1.22 (3H, t, J=7.3 Hz), 1.26 (3H, d, J=5.9 Hz), 3.47—4.20 (4H, m), 4.04, 5.11 (2H, ABq, J=12.4 Hz), 4.11 (2H, q, J=7.3 Hz), 4.65 (1H, q, J=5.9 Hz), 5.07 (1H, d, J=4.0 Hz), 5.55 (1H, d, J=7.3 Hz), 5.71 (1H, dd, J=3.7 9.5 Hz), 6.26 (1H, s), 6.58 (1H, s), 6.88 (1H, s), 7.20—7.60 (25H, m), 7.85 (1H, d, J=9.5 Hz), 9.75 (1H, d, J=7.4 Hz).

The compound of Rf 0.26 shows:

NMR ($CDCl_3$), (ppm): 1.21 (3H, t, J=7.1 Hz), 1.39 (3H, d, J=6.6 Hz) 3.50—4.18 (4H, m) 4.04, 5.16 (2H, ABq, J=12.4 Hz), 4.11 (2H, q, J=7.1 Hz), 4.73 (1H, q, J=6.6 Hz), 4.75 (1H, d, J=4.0 Hz), 5.50 (1H, d, J=6.9 Hz), 5.64 (1H, dd, J=4.0, 9.1 Hz), 6.29 (1H, s), 6.58 (1H, s), 6.87 (1H, s), 7.20—7.50 (25H, m), 7.57 (1H, d, J=9.1 Hz), 9.77 (1H, d, J=6.9 Hz).

(6) The following steps are described for the isomer compound of Rf 0.43, but are equally applicable to the isomer compound of Rf 0.26.

113 mg of the isomer compound (Rf=0.43) mentioned above, 23 mg of 2,5-dihydro-6-hydroxy-2-methyl-3-mercapto-5-oxo-1,2,4-triazine sodium salt and 17 mg of NaI were dissolved in 0.5 ml of DMF. The solution obtained was agitated at ambient temperature for 2 hours.

The reaction solution was diluted with ethyl acetate and washed with acidified water (pH 3.0) and then with water, dried over $MgSO_4$ and concentrated.

The residue was purified by column chromatography on silica gel developed with a development solvent of chloroform-methanol (20:1) to give 115 mg of diphenylmethyl 7β - [2 - (4 - ethyl - 2,3 - dioxo - 1 - piperazinecarboxamido) - 2 - (2 - tritylaminothiazol - 4 - yl)acetamido] - 2β - methyl - 3 - (2,5 - dihydro - 6 - hydroxy - 2 - methyl - 5 - oxo - 1,2,4 - triazin - 3 - yl)thiomethyl - 1 - oxa - 1 - dethia - 3 - cephem - carboxylate.

(7) 115 Mg of the product from the step (6) above was added to a mixture of trifluoroacetic acid (1.2 ml) and anisol (0.2 ml) under ice-cooling, and the resulting mixture was agitated at the same temperature as above for 30 minutes. To the reaction mixture was added IPE to deposit a precipitate which was then removed by filtration.

The precipitate obtained by filtration and sodium bicarbonate (35 mg) were dissolved in 1 ml of water and purified by column chromatography on silica gel developed with water to give disodium salt of the title compound which shows the following physicochemical properties.

IR (KBr), $_{max}$($cm^{-1}$): 1765, 1710, 1675, 1610

NMR($D_2O$), (ppm): 1.18 (3H, t, J=7.4 Hz), 1.35 (3H, d, J=6.6 Hz), 3.30—4.10 (4H, m), 3.50 (2H, q, J=7.4 Hz), 3.61 (3H, s), 3.94, 4.43 (2H, ABq, J=13.3 Hz), 5.12 (1H, d, J=4.6 Hz), 5.42 (1H, s), 5.43 (1H, d, J=4.6 Hz), 6.76 (1H, s).

On the other hand, disodium (6R, 7R) - 7 - [2 - (2 - aminothiazol - 4 - yl) - 2 - (4 - ethyl - 2,3 - dioxo - 1 - piperazine-carboxamido)acetamido] - (2S) - 2 - methyl - 3 - (2,5 - dihydro - 6 - hydroxy - 2 - methyl - 5 - oxo - 1,2,4 - triazin - 3 - yl)thiomethyl - 1 - oxa - 1 - dethia - 3 - cephem - 4 - carboxylate (disodium salt of compound No. 1) was obtained in the same manner as mentioned above for the compound Rf 0.43 from the isomer compound of Rf 0.26, and it shows the following physicochemical properties:

IR (KBr), $_{max}$($cm^{-1}$): 1763, 1710, 1678, 1610

NMR($D_2O$), (ppm): 1.18 (3H, t, J=7.3 Hz), 1.42 (3H, d, J= 6.6 Hz), 3.30—4.10 (4H, m), 3.50 (2H, q, J=7.3 Hz), 3.62 (3H, s), 3.94, 4.44 (2H, ABq, J=13.9 Hz), 5.17 (1H, d, J=3.6 Hz), 5.37 (1H, d, J=3.6 Hz), 5.43 (1H, s), 6.74 (1H, s).

Example 2

Production of disodium (6R, 7R) - 7 - [2 - (2 - aminothiazol - 4 - yl) - 2 - (formamido)acetamido] - (2S) - 2 - methyl - 3 - (2,5 - dihydro - 6 - hydroxy - 2 - methyl - 5 - oxo - 1,2,4 - triazin - 3 - yl)-thiomethyl - 1 - oxa - 1 - dethia - 3 - cephem - 4 - carboxylate - (disodium salt of compound No. 2)

(1) 130 Mg of diphenylmethyl 7β-amino-2β-methyl-3-chloromethyl-1-oxadethia-3-cephem-4-carboxylate hydrochloride and 136 mg of sodium DL-2-(2-trityl-aminothiazol-4-yl)-2-formamido-acetate were dissolved in 10 ml of methylene chloride. To the solution obtained were added 105 μl of pyridine and 35 μl of phosphorous oxychloride at −20°C, and the mixture was agitated at the same temperature as above for 30 minutes and then under ice-cooling for 30 minutes.

The reaction solution was washed with acidified water (pH 3.0), with saturated aqueous sodium bicarbonate and then with water, dried over $MgSO_4$ and concentrated.

The residue was purified by column chromatography on silica gel developed with a development solvent of toluene-ethyl acetate (1:1) to give 160 mg of diphenylmethyl 7β-[DL-2-(2-tritylaminothiazol-4-yl)-2-(formamido)acetamido]-2β-methyl-3-chloromethyl-1-oxa-1-dethia-3-cephem-4-carboxylate.

IR ($CDCl_3$), $_{max}(cm^{-1})$: 1776, 1725, 1660

NMR($CDCl_3$), (ppm): 1.27, 1.38 (3H, d), 4.05, 5.13 (2H, ABq, 4.66, 4.73 (1H, q), 4.95, 5.07 (1H, d), 5.50, 5.55 (1H, d), 5.64, 5.71 (1H, dd), 6.29 (1H, s), 6.58 (1H, s), 6.88 (1H, s), 7.20—7.60 (25H, m), 8.34 (1H, s), 9.77 (1H, d).

(2) 160 Mg of the product from the step (1) above, 41 mg of 2,5-dihydro-6-hydroxy-2-methyl-3-mercapto-5-oxo-1,2,4-triazine sodium salt and 34 mg of sodium iodide were dissolved in 1 ml of DMF, and the solution was agitated at ambient temperature of 1 hour.

The reaction solution was diluted with 10 ml of ethyl acetate and washed with acidified water (pH 3.0 and then with water, dried over $MgSO_4$ and concentrated.

The residue was purified by column chromatography on silica gel developed with chloroform-methanol (10:1) to give 150 mg of diphenylmethyl 7β - [DL - 2 - (2 - tritylaminothiazol - 4 - yl) - 2 - (formamido) acetamido] - 2β - methyl - 3 - (2,5 - dihydro - 6 - hydroxy - 2 - methyl - 5 - oxo - 1,2,4 - triazin - 3 - yl )thiomethyl - 1 - oxadethia - 3 - cephem - 4 - carboxylate.

(3) 150 Mg of the product from the step (2) above was added to a mixture of trifluoroacetic acid (2 ml) and anisol (0.2 ml) under ice-cooling, and the mixture obtained was agitated at the same temperature as above for 30 minutes, to which then 10 ml of IPE was added to deposit a precipitate which was removed by filtration.

The precipitate (100 mg) obtained and sodium bicarbonate (50 mg) were dissolved in 1 ml of water and purified by column chromatography on Diaion HP—20 developed with water to give 55 mg of disodium salt of the titled compound (Compound No. 6).

NMR($D_2O$), (ppm): 1.39, 1.45 (3H, d), 3.61 (3H, s), 3.81, 4.42 (2H, ABq), 5.12, 5.16 (1H, d), 5.37, 5.43 (1H, d), 5.42, 5.43 (1H, s), 6.76 (1H, s), 8.18 (1H, s).

## Claims

1. A 1-oxa-1-dethia-cephalosporin compound represented by the general formula (I)

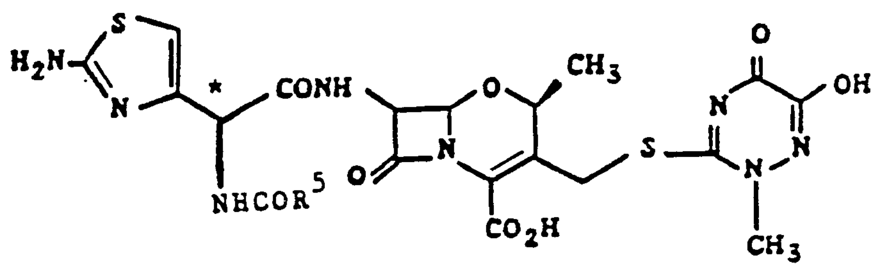

where $R^5$ is a hydrogen atom, a 4-ethyl-2,3-dioxo-piperazin-1-yl group or is selected from the groups consisting of:

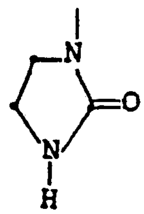 , 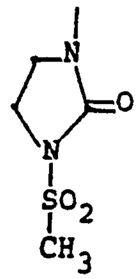 , 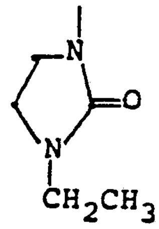 , 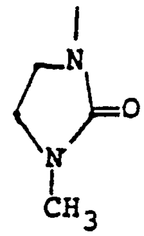 , 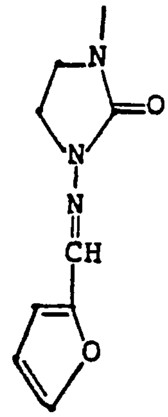

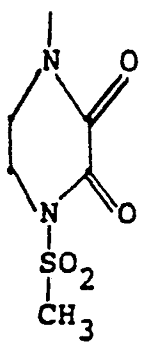 , 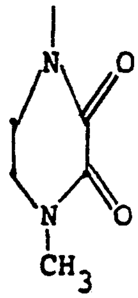 or 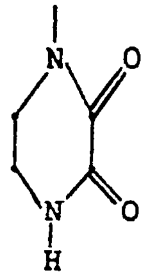

and the carbon atom to which the asterisk is attached shows (R)-configuration or (S)-configuration or a mixture of these configurations; and a pharmaceutically acceptable hydrate, salt or ester thereof.

2. A pharmaceutical composition for antibacterial use comprising an antibacterially effective amount of a compound according to Claim 1 and a pharmaceutically acceptable carrier.

3. A 1-oxo-1-dethia-cephalosporin compound represented by the general formula (IIb)

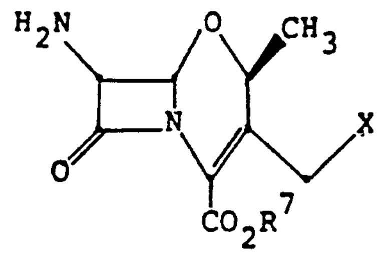

in which X is a chlorine atom, a bromine atom or an iodine atom, and $R^7$ is a hydrogen atom or a carboxyl-protecting group.

**Patentansprüche**

1. Eine 1-Oxa-1-dethia-cephalosporinverbindung wiedergegeben durch die allgemeine Formel (I)

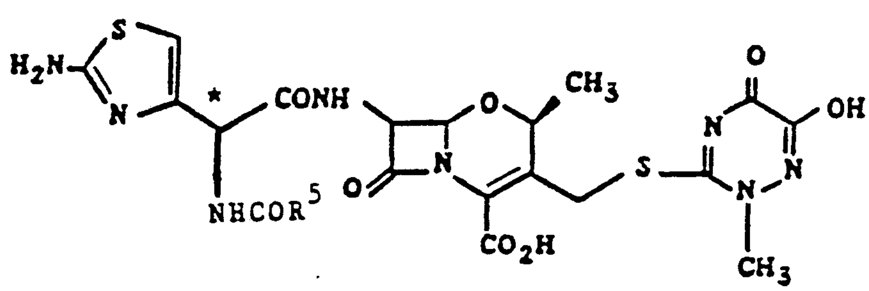

worin $R^5$ ein Wasserstoffatom, eine 4-Ethyl-2,3-dioxopiperazin-1-yl-Gruppe ist oder ausgewählt ist aus den Gruppen bestehend aus:

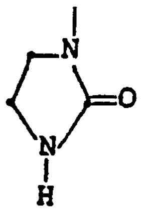, 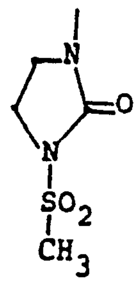, 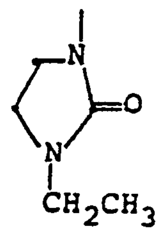, 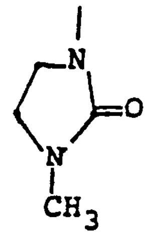, 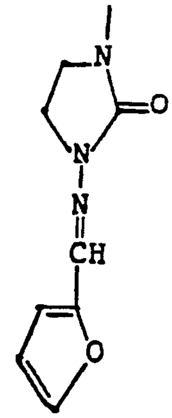

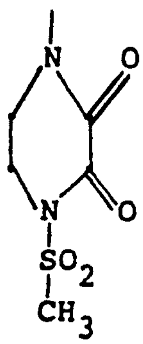, 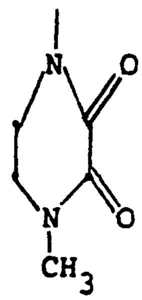 oder 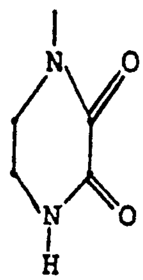

und worin das mit einem Sternchen versehene Kohlenstoffatom (R)- oder (S)-Konfiguration oder eine Mischung dieser Konfigurationen zeigt, und ein pharmazeutisch annehmbares Hydrat oder Salz oder ein pharmazeutisch annehmbarer Ester davon.

2. Eine pharmazeutische Zusammensetzung für antibakterielle Verwendung, enthaltend eine antibakteriell wirksame Menge einer Verbindung gemäss Anspruch 1 und einen pharmazeutisch annehmbaren Träger.

3. Eine 1-Oxo-1-dethia-cephalosporinverbindung, wiedergegeben durch die allgemeine Formel (IIb)

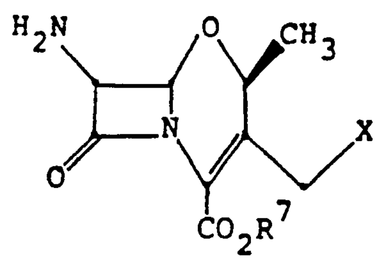

worin X ein Chloratom, ein Bromatom oder ein Iodatom ist und worin $R^7$ ein Wasserstoffatom oder eine Carboxylschutzgruppe ist.

**Revendications**

1. Dérivé de 1-oxa-1-déthia-céphalosporine représenté par la formule générale (I)

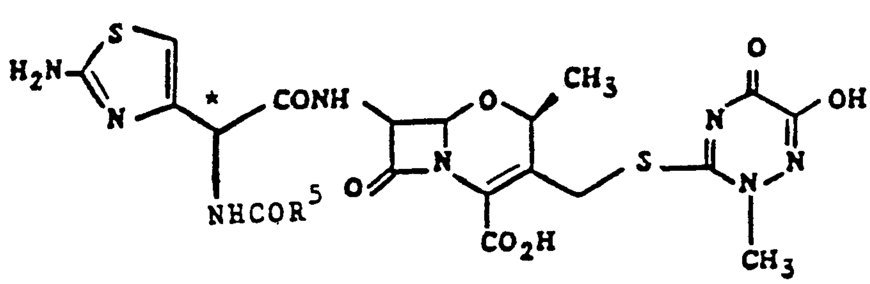

dans laquelle $R^5$ est un atome d'hydrogène, un groupe 4-éthyl-2,3-dioxo-1-pipérazine-1-yle ou est choisi dans le groupe constitué de:

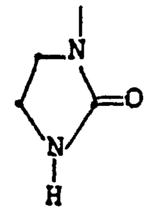 , 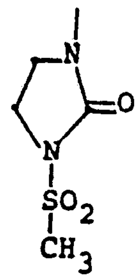 , 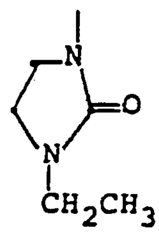 , 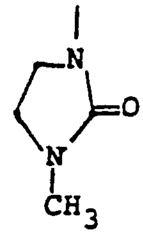 , 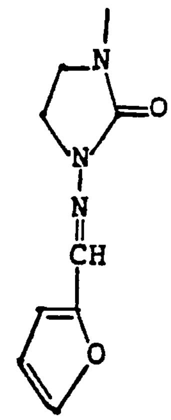

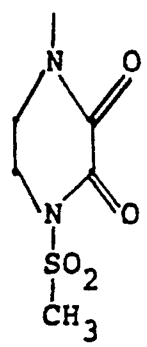 , 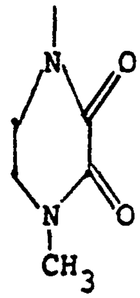 ou 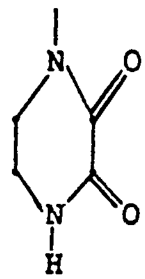

et l'atome de carbone auquel l'astérisque est fixé présente la configuration R ou la configuration S ou un mélange de ces configurations; et un hydrate, sel ou ester pharmaceutiquement acceptables de celui-ci.

2. Composition pharmaceutique à usage antibactérien comprenant une quantité antibactérienne efficace d'un composé selon la revendication 1 et un véhicule pharmaceutiquement acceptable.

3. Dérivé de 1-oxo-1-déthia-céphalosporin représenté par la formule générale (IIb)

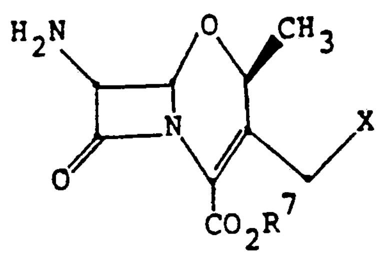

dans laquelle X est un atome de chlore, un atome de brome ou un atome d'iode, et $R^7$ est un atome d'hydrogène ou un groupe carboxy-protecteur.